# EUROPEAN PATENT APPLICATION

(11) **EP 4 364 747 A1**
(43) Date of publication of application: **08.05.2024**
(21) Application number: 22833260.7
(22) Date of filing: 30.06.2022
(51) Int. Cl.: A61K 35/28, A61P 9/00, A61P 9/04, A61P 9/10, C12N 5/0789

(54) **PHARMACEUTICAL COMPOSITION CONTAINING IRRADIATED HEMATOPOIETIC STEM CELLS**

(30) Priority: 02.07.2021 JP 2021110601
(71) Applicant: Foundation for Biomedical Research and Innovation at Kobe, Kobe-shi, Hyogo 650-0047 (JP); Japanese Red Cross Society, Tokyo 105-8521 (JP)
(72) Inventor: TAGUCHI Akihiko, Kobe-shi, Hyogo 650-0047 (JP); TAURA Akie, Kobe-shi, Hyogo 650-0047 (JP); OKINAKA Yuka, Kobe-shi, Hyogo 650-0047 (JP); OGAWA Yuko, Kobe-shi, Hyogo 650-0047 (JP); KIMURA Takafumi, Ibaraki City, Osaka 5670085 (JP); TANAKA Mitsunobu, Ibaraki City, Osaka 5670085 (JP); YASUI Kazuta, Ibaraki City, Osaka 5670085 (JP); Fuchizaki Akihiro, Ibaraki City, Osaka 5670085 (JP); YASUI Teruhito, Ibaraki City, Osaka 5670085 (JP)
(74) Representative: J A Kemp LLP
(86) International application number: PCT/JP2022/026147
(87) International publication number: WO 2023/277114

(57) **Abstract**

The present invention relates to a pharmaceutical composition comprising a population of cells including irradiated hematopoietic stem cells, and a method for producing the pharmaceutical composition, specifically, a pharmaceutical composition for treating an ischemic disease, comprising a population of cells including irradiated hematopoietic stem cells, wherein the irradiation is irradiation at 50 Gy or less, and a method for producing the pharmaceutical composition.

## Description

### Technical Field

### [Related Application]

The present application claims priority to Japanese Patent Application No. 2021-110601 (filed on July 2, 2021), the disclosure of which is incorporated herein by reference.

### [Technical Field]

The present invention relates to a pharmaceutical composition comprising a population of cells including irradiated hematopoietic stem cells, and a method for producing the pharmaceutical composition.

### Background Art

For age-related macular degeneration, transplantation of retinal pigment epithelial cells derived from iPS cells (artificial multipotent stem cells) is applied, but at all times, there is the risk of tumor formation or canceration caused by residual undifferentiated cells. On the other hand, it is believed that stem cells should not be exposed to radiation that causes DNA damage because engraftment of the transplanted stem cells is required for stem cell transplantation.

Patients with blood disease undergo hematopoietic stem cell transplantation therapies with allogeneic bone marrow or umbilical cord blood, but lymphocyte-induced adverse immune reactions or graft versus host diseases (GVHDs) occur with high frequency as allogeneic immune cells and hematopoietic stem cells enter the bodies of the recipients. On the other hand, it is generally understood that since irradiation causes DNA cleavage and loss of proliferative ability of hematopoietic stem cells, cells to be administered should not be irradiated when transplanting hematopoietic stem cells into a blood disease patient.

A case has been reported in which critical limb ischemia is treated with apoptotic hematopoietic stem cells induced by irradiation at 70 Gy. In this case, cells immediately after irradiation are transplanted because nearly 70% of the cells die in 48 hours after irradiation (Non-Patent Literature 1).

In hematopoietic stem cell therapies, the problems of greatest concern from the viewpoint of safety are (i) transplanted cell-induced adverse immune reactions, (ii) graft versus host diseases and (iii) canceration of transplanted cells. These problems are known to lead to a lethal outcome. However, as described above, irradiation performed for suppressing immune reactions and canceration significantly reduces the cell viability, and therefore therapies using irradiated cells have not been popular.

### Citation List

### Non-Patent Literature

Non-Patent Literature 1: Perotti et al., Allogeneic lethally irradiated cord blood mononuclear cells in no-option critical limb ischemia: a "box of rain" Stem Cells Dev. 2013 Oct 15; 22(20): 2806-12.

### Summary of Invention

### Technical Problem

An object of the present invention is to provide a safe and effective cell therapy with hematopoietic stem cells by solving the problems of the conventional art.

### Solution to Problem

The present inventors have performed irradiation under specific conditions to successfully prepare cells that do not undergo a decrease in cell viability over time, and are not proliferative.

That is, in a first aspect, the present invention provides the following [1] to [14].
[1] A pharmaceutical composition, preferably a pharmaceutical composition for treating an ischemic disease, comprising a population of cells including irradiated hematopoietic stem cells, wherein the irradiation is irradiation at 50 Gy or less.
   The irradiation is irradiation at preferably 45 Gy or less, more preferably 40 Gy or less, further more preferably 35 Gy or less, particularly preferably 30 Gy or less.
[2] The pharmaceutical composition according to [1], wherein the population of cells has cell viability of 90% or more even 3 hours after the irradiation.
[3] The pharmaceutical composition according to [1] or [2], wherein the population of cells including hematopoietic stem cells is a population of mononuclear cells.
[4] The pharmaceutical composition according to any one of [1] to [3], wherein the population of cells including hematopoietic stem cells is a population of CD34-positive cells.
[5] The pharmaceutical composition according to any one of [1] to [4], wherein the population of cells including hematopoietic stem cells is derived from umbilical cord blood, bone marrow liquid or peripheral blood.
[6] The pharmaceutical composition according to any one of [1] to [5], wherein the irradiation is irradiation at 7.5 Gy to 30 Gy. The irradiation is irradiation at preferably 10 Gy to 30 Gy, more preferably 15 Gy to 30 Gy.
[7] The pharmaceutical composition according to any one of [1] to [6], wherein the ischemic disease is one selected from the group consisting of limb ischemia, myocardial infarction, angina pectoris, cardiac failure, brain infarction, arterial dissection, venous infarction, vasculitis, vascular dementia, cerebral palsy including neonatal hypoxic ischemic encephalopathy, bilirubin encephalopathy at premature birth, and refractory bone fracture associated with an impaired blood flow.
[8] The pharmaceutical composition according to [6], wherein the ischemic disease is brain infarction.
[9] A method for producing a pharmaceutical composition, preferably a pharmaceutical composition for treating an ischemic disease, the method comprising the steps of:
   1) preparing a population of cells including irradiated hematopoietic stem cells by one of the following methods (a) and (b):
      (a) irradiating a sample containing hematopoietic stem cells at 50 Gy or less, and isolating a population of cells including hematopoietic stem cells from the sample, and
      (b) isolating a population of cells including hematopoietic stem cells from a sample containing hematopoietic stem cells, and irradiating the population of cells at 50 Gy or less; and
   2) producing a pharmaceutical composition comprising the population of cells including irradiated hematopoietic stem cells.
      The irradiation is irradiation at preferably 45 Gy or less, more preferably 40 Gy or less, further more preferably 35 Gy or less, particularly preferably 30 Gy or less.
[10] The method according to [10], wherein irradiation at 7.5 Gy to 30 Gy is performed. The irradiation is irradiation at preferably 10 Gy to 30 Gy, more preferably 15 Gy to 30 Gy.
[11] The method according to [9] or [10], wherein the population of cells including hematopoietic stem cells is a population of mononuclear cells.
[12] The method according to any one of [9] to [11], wherein the population of cells including hematopoietic stem cells is a population of CD34-positive cells.
[13] The method according to any one of [9] to [12], wherein the population of cells including hematopoietic stem cells is derived from umbilical cord blood, bone marrow liquid or peripheral blood.
[14] The method according to any one of [9] to [13], comprising the step of adding a cryoprotectant to the pharmaceutical composition, followed by freezing.

In a second aspect, the present invention provides the following [1] to [17].
[1] A method for treating a subject, preferably a subject with an ischemic disease, the method comprising administering a population of cells including irradiated hematopoietic stem cells to the subject, wherein the irradiation is irradiation at 50 Gy or less.
[2] The method according to [1], wherein the population of cells has cell viability of 90% or more even 3 hours after the irradiation.
[3] The method according to [1], wherein the population of cells including hematopoietic stem cells is a population of mononuclear cells.
[4] The method according to [1], wherein the population of cells including hematopoietic stem cells is a population of CD34-positive cells.
[5] The method according to [1], wherein the population of cells including hematopoietic stem cells is derived from umbilical cord blood, bone marrow liquid or peripheral blood.
[6] The method according to [1], wherein the irradiation is irradiation at 7.5 Gy to 30 Gy.
[7] The method according to [1], wherein the ischemic disease is one selected from the group consisting of limb ischemia, myocardial infarction, angina pectoris, cardiac failure, brain infarction, arterial dissection, venous infarction, vasculitis, vascular dementia, cerebral palsy including neonatal hypoxic ischemic encephalopathy, bilirubin encephalopathy at premature birth, and refractory bone fracture associated with an impaired blood flow.
[8] The method according to [1], wherein the ischemic disease is brain infarction.
[9] The method according to [1], wherein the population of cells including hematopoietic stem cells is administered into a vein or an artery.
[10] The method according to [1], wherein the population of cells including hematopoietic stem cells is administered into a carotid artery.
[11] The method according to [1], wherein a risk of inducing an immune reaction after transplantation (for example, a risk of developing a graft versus host disease) is lower as compared to a population of non-irradiated hematopoietic stem cells.
[12] A method for producing a cell preparation, preferably a cell preparation for treating an ischemic disease, the method comprising the steps of:
   1) preparing a population of cells including irradiated hematopoietic stem cells by one of the following methods (a) and (b):
      (a) irradiating a sample containing hematopoietic stem cells at 50 Gy or less, and isolating a population of cells including hematopoietic stem cells from the sample, and
      (b) isolating a population of cells including hematopoietic stem cells from a sample containing hematopoietic stem cells, and irradiating the population of cells at 50 Gy or less; and
   2) producing a cell preparation comprising the population of cells including irradiated hematopoietic stem cells, and a pharmacologically acceptable carrier.
[13] The method according to [12], wherein irradiation at 10 Gy to 30 Gy is performed.
[14] The method according to [12], wherein the population of cells including hematopoietic stem cells is a population of mononuclear cells.
[15] The method according to [12], wherein the population of cells including hematopoietic stem cells is a population of CD34-positive cells.
[16] The method according to [12], wherein the population of cells including hematopoietic stem cells is derived from umbilical cord blood, bone marrow liquid or peripheral blood.
[17] The method according to [12], comprising the step of adding a cryoprotectant to the cell preparation, followed by freezing.

In a third aspect, the present invention provides the following [1] to [15].
[1] A population of cells comprising irradiated hematopoietic stem cells, for use in the method of treating a subject, preferably a subject with an ischemic disease, wherein the irradiation is irradiation at a dose which allows the maintenance of cell viability equivalent to that of non-irradiated counterparts during a period necessary for therapeutic efficacy, specifically, 50 Gy or less, preferably 45 Gy or less, more preferably 40 Gy or less, further more preferably 35 Gy or less, particularly preferably 30 Gy or less.
[2] The population of cells for use according to [1], wherein the population of cells has cell viability of 90% or more even 3 hours after the irradiation.
[3] The population of cells for use according to [1] or [2], wherein the population of cells including hematopoietic stem cells is a population of mononuclear cells.
[4] The population of cells for use according to any one of [1] to [3], wherein the population of cells including hematopoietic stem cells is a population of CD34-positive cells.
[5] The population of cells for use according to any one of [1] to [4], wherein the population of cells including hematopoietic stem cells is derived from umbilical cord blood, bone marrow liquid or peripheral blood.
[6] The population of cells for use according to any one of [1] to [5], wherein the irradiation is irradiation at 7.5 Gy to 30 Gy, preferably 10 Gy to 30 Gy, more preferably 15 Gy to 30 Gy.
[7] The population of cells for use according to any one of [1] to [6], wherein the ischemic disease is one selected from the group consisting of limb ischemia, myocardial infarction, angina pectoris, cardiac failure, brain infarction, arterial dissection, venous infarction, vasculitis, vascular dementia, cerebral palsy including neonatal hypoxic ischemic encephalopathy, bilirubin encephalopathy at premature birth, and refractory bone fracture associated with an impaired blood flow, preferably brain infarction.
[8] The population of cells for use according to any one of [1] to [7], wherein the population of cells including hematopoietic stem cells is administered into a vein or an artery.
[9] The population of cells for use according to any one of [1] to [8], wherein the population of cells including hematopoietic stem cells is administered into a carotid artery.
[10] The population of cells for use according to any one of [1] to [9], wherein a risk of inducing an immune reaction after transplantation (for example, a risk of developing a graft versus host disease) is lower as compared to a population of non-irradiated hematopoietic stem cells.
[11] A method for producing a cell preparation, preferably a cell preparation for treating an ischemic disease, the method comprising the steps of:
   1) preparing a population of cells including irradiated hematopoietic stem cells by one of the following methods (a) and (b):
      (a) irradiating a sample containing hematopoietic stem cells at 50 Gy or less, and isolating a population of cells including hematopoietic stem cells from the sample, and
      (b) isolating a population of cells including hematopoietic stem cells from a sample containing hematopoietic stem cells, and irradiating the population of cells at 50 Gy or less; and
   2) producing a cell preparation comprising the population of cells including irradiated hematopoietic stem cells, and a pharmacologically acceptable carrier.
[12] The method according to [11], wherein irradiation at 7.5 Gy to 30 Gy, preferably 10 Gy to 30 Gy, more preferably 15 Gy to 30 Gy is performed.
[13] The method according to [11] or [12], wherein the population of cells including hematopoietic stem cells is a population of mononuclear cells, preferably a population of mononuclear cells derived from umbilical cord blood, bone marrow liquid or peripheral blood.
[14] The method according to any one of [11] to [13], wherein the population of cells including hematopoietic stem cells is a population of CD34-positive cells, preferably a population of CD34-positive cells derived from umbilical cord blood, bone marrow liquid or peripheral blood.
[15] The method according to any one of [11] to [14], comprising the step of adding a cryoprotectant to the cell preparation, followed by freezing.

### Advantageous Effect of Invention

The present invention provides a population of cells including hematopoietic stem cells which ensures that (i) transplanted cell-induced adverse immune reactions are avoided, (ii) there is no risk of developing a graft versus host disease, (iii) there is no possibility of canceration of transplanted cells, and (iv) the cells have a good cell viability. Since the population of cells has the advantages described above, it can be incorporated a stem cell bank, and is useful for cell therapies with hematopoietic stem cells.

### Brief Description of Drawings

[Figure 1] Figure 1 shows graphs showing the proportion of viable cells (Annexin V-negative and 7AAD-negative cells) in CD34-positive cells, T cells (CD3-positive), B cells (CD19-positive) and monocytes (CD14-positive).
[Figure 2] Figure 2 shows graphs showing the proportion of viable cells (Annexin V-negative and 7AAD-negative cells) 4 hours after irradiation. From the left, hematopoietic stem cells (CD34-positive cells), T cells (CD3-positive cells), B cells (CD19-positive cells) and monocytes (CD14-positive cells).
[Figure 3] Figure 3 shows graphs showing the proportion of viable cells (Annexin V-negative and 7AAD-negative cells) 48 hours after irradiation. From the left, hematopoietic stem cells (CD34-positive cells), T cells (CD3-positive cells), B cells (CD19-positive cells) and monocytes (CD14-positive cells).
[Figure 4] Figure 4 shows graphs showing the results of a lymphocyte stimulation test with PHA: the number of T cells on days 1, 7 and 10 with day 0 as a start point. A: non-irradiated cells, B: irradiated cells
[Figure 5] Figure 5 shows graphs showing the results of a colony formation test: the number of colonies after culturing for 14 days. A: non-irradiated cells, B: irradiated cells, In each case, 1 × 10² cells, 1 × 10³ cells and 1 × 10⁴ cells are seeded.
[Figure 6] Figure 6 is a graph showing the amount (%) of VEGF captured in vascular endothelial cells. Left: non-irradiated cells, right: irradiated cells
[Figure 7] Figure 7 is a graph showing the results of a wire hang test: a time (sec) until a mouse falls down from a wire. From the left, no-surgery group, PBS administration group, MNC administration group, MNC-X administration group, CD34⁺ administration group and CD34⁺-X administration group. From the left, no-surgery group, PBS administration group, MNC administration group, MNC-X administration group, CD34⁺ administration group and CD34⁺-X administration group.
   No-surgery group and PBS administration group: n = 6, MNC administration group, MNC-X administration group, CD34⁺ administration group and CD34⁺-X administration group: *p < 0.05, **p < 0.01
[Figure 8] Figure 8 is a graph showing the results of a rotarod test: a time (sec) until a mouse falls down from a rotation bar after being placed thereon. From the left, no-surgery group, PBS administration group, MNC administration group, MNC-X administration group, CD34⁺ administration group and CD34⁺-X administration group. No-surgery group and PBS administration group: n = 6, MNC administration group, MNC-X administration group, CD34⁺ administration group and CD34⁺-X administration group: n = 5, *p < 0.05, **p < 0.01, ***p < 0.001
[Figure 9] Figure 9 is a graph showing the results of an open field test: a total count of beam interrupts. From the left, no-surgery group, PBS administration group, MNC administration group, MNC-X administration group, CD34⁺ administration group and CD34⁺-X administration group. No-surgery group and PBS administration group: n = 6, MNC administration group, MNC-X administration group, CD34⁺ administration group and CD34⁺-X administration group: n = 5, *p < 0.05, **p < 0.01
[Figure 10] Figure 10 shows graphs showing the concentrations of low-molecular energy sources before and after irradiation. Left: glucose, center: glutamate, right: lactate
[Figure 11] Figure 11 shows graphs showing the results of a lymphocyte stimulation test with anti-CD3/CD28 antibody-bound beads and IL-2: the amplification ratio (times) of T cells on days 1, 4, 6 and 10 with day 0 as a start point. A: non-irradiated cells, B: irradiated cells
[Figure 12] Figure 12 shows graphs showing the ratio of human CD45⁺ cells in the peripheral blood of immunodeficient mice (NOG-SCID mice) 4 and 8 weeks after transplantation. A: non-irradiated cells, B: irradiated cells
[Figure 13] Figure 13 shows graphs showing the proportion (%) of viable cells in non-irradiated cells (left) and irradiated cells (right) after lymphocyte stimulation. A: T cells and B: CD34⁺ cells.
[Figure 14] Figure 14 is a graph showing the amplification ratio of T cells when the dose of an X-ray used for irradiation is changed.

### Description of Embodiments

### 1. Population of cells including irradiated hematopoietic stem cells (XR cells)

The present invention relates to a population of cells including irradiated hematopoietic stem cells (hereinafter, sometimes referred to "XR cells"), and use of the population of cells as a pharmaceutical composition.

### 1.1 Population of cells including hematopoietic stem cells

In the present invention, the "hematopoietic stem cell" is a multipotent stem cell that can differentiate into hematopoietic cells. The "hematopoietic stem cell" is characterized by being CD34-positive, exists in the bone marrow, peripheral blood, placenta and umbilical cord blood and the like of adults, and is contained in a relatively high proportion, especially in mononuclear cell fractions. The hematopoietic stem cells according to the present invention also include CD34-positive progenitor cells which are derived from hematopoietic stem cells, and which have not terminally differentiated (sometimes referred to as hematopoietic progenitor cells).

In the present invention, the "population of cells including hematopoietic stem cells" is a population of cells which is enriched for hematopoietic stem cells, and includes hematopoietic stem cells in an amount of preferably 0.05% or more, more preferably 0.3% or more, further more preferably 1% or more, particularly preferably 3% or more.

The origin of the "population of cells including hematopoietic stem cells" is not limited, and examples thereof include bone marrow, peripheral blood, placenta, and umbilical cord blood. Preferably, the "population of cells including hematopoietic stem cells" is derived from bone marrow, peripheral blood or umbilical cord blood, more preferably umbilical cord blood.

As the "population of cells including hematopoietic stem cells", a "population of mononuclear cells" and a "population of CD34-positive cells" in which hematopoietic stem cells are included in a relatively high proportion may be used. The "population of mononuclear cells" can be obtained from a sample of bone marrow, peripheral blood, placenta, umbilical cord blood or the like by density gradient centrifugation according to a method well known in the art.

The "population of CD34-positive cells" means a population of cells enriched for CD34-positive, and can be obtained from a sample of bone marrow, peripheral blood, placenta, umbilical cord blood or the like by concentrating cells for the CD34-positive according to a method well known in the art. The concentration of CD34-positive cells can be performed using CD34 immobilized beads or a cell sorter. A preferred example of CD34-positive cells is a population of CD34-positive mononuclear cells obtained by performing concentration of CD34-positive cells on a population of mononuclear cells.

The "population of cells including hematopoietic stem cells" can also be obtained using a known hematopoietic stem cell marker. In addition to CD34-positive, known markers characterizing hematopoietic stem cells include CD38-negative, CD90-positive, CD45RA-negative, and CD49f-positive for human hematopoietic stem cells. Therefore, the "population of cells including hematopoietic stem cells" can be obtained from a sample containing hematopoietic stem cells of bone marrow, peripheral blood, placenta, umbilical cord blood or the like by concentrating hematopoietic stem cells using one or more of these markers. Therefore, the "population of cells including hematopoietic stem cells" can be obtained from a sample containing hematopoietic stem cells from bone marrow, peripheral blood, placenta, umbilical cord blood or the like by concentrating hematopoietic stem cells using one or more of these markers.

The "population of cells including hematopoietic stem cells" according to the present invention is intended to use allogeneic cells for the purpose of irradiation, but does not exclude autologous cells. In one embodiment, the "population of cells including hematopoietic stem cells" is prepared from an allogeneic umbilical cord blood sample, in the pharmaceutical composition of the present invention. In another embodiment, the "population of cells including hematopoietic stem cells" is prepared from a cell population in a cell bank.

### 1.2 Irradiation

The "irradiation" used in the present invention suppresses proliferation of cells, but allows maintenance of cell viability equivalent to that of non-irradiated counterparts for a period of time necessary for therapeutic efficacy.

The radiation is not limited as long as it is used for damaging DNA of cells in medical treatment, and may be an X-ray, a γ-ray, a heavy particle beam or the like, with an X-ray being preferred.

The dose of the radiation is, for example, 50 Gy, 45 Gy, 40 Gy, 35 Gy, 30 Gy, 25 Gy, 20 Gy or 18 Gy for the upper limit, and 10 Gy, 12 Gy, 13 Gy, 14 Gy or 15 Gy for the lower limit, and is in the range of, for example, 7.5 to 50 Gy, 10 to 50 Gy, 10 to 30 Gy, 15 to 50 Gy, 15 to 30 Gy or 15 to 25 Gy. The dose is preferably 7.5 to 30 Gy, more preferably 10 Gy to 30 Gy, more preferably 15 to 30 Gy, further more preferably 15 to 25 Gy. For example, irradiation at 15 Gy, preferably an X-ray, is performed.

The irradiation may precede or follow the separation/concentration of cells. For example, a sample containing hematopoietic stem cells from bone marrow, peripheral blood, placenta, umbilical cord blood or the like may be irradiated, followed by separation of a "population of cells including hematopoietic stem cells", or a "population of cells including hematopoietic stem cells" may be separated from a sample, followed by irradiation of the population of cells.

### 1.3 Characteristics of XR cells

The "XR cells" according to the present invention can maintain cell viability equivalent to that of non-irradiated cells for 4 hours or more, preferably 10 hours or more, more preferably 24 hours or more, particularly preferably 48 hours or more after irradiation. Specifically, the "XR cells" can maintain cell viability of 90% or more for 4 hours or more, preferably 10 hours or more, more preferably 24 hours or more, particularly preferably 48 hours or more after irradiation.

The "XR cells" according to the present invention are not proliferative because their proliferative mitotic activity is suppressed. When cultured *in vitro,* the "XR cells" die after 20 days at most, preferably 15 days, more preferably 12 days, particularly preferably 10 days. Therefore, the "XR cells" according to the present invention have no risk of canceration after transplantation.

The "XR cells" according to the present invention are not immunoreactive to antigen stimulation, and unlikely to induce an immune reaction after transplantation. Therefore, the "XR cells" have little risk of developing a graft versus host disease. The "XR cells" become apoptotic only after receiving proliferation signals, so that a high viability is maintained during a period necessary for therapeutic efficacy.

The "XR cells" according to the present invention comprise hematopoietic stem cells, and are comparable in revascularization promotion ability to non-irradiated cells.

### 2. Treatment of ischemic disease and the like

### 2.1 Pharmaceutical composition (cell preparation)

As described above, the XR cells according to the present invention have hematopoietic stem cell ability equivalent to that of the non-irradiated counterparts, such that (i) transplanted cell-induced adverse immune reactions are avoided, (ii) there is no risk of developing a graft versus host disease, (iii) there is no possibility of canceration of transplanted cells, (iv) the cells have a good cell viability, and (v) ability to promote revascularization is exhibited. Therefore, the XR cells according to the present invention can be used as a pharmaceutical composition (cell preparation) for ischemic diseases.

The pharmaceutical composition of the present invention includes a cell population including irradiated hematopoietic stem cells, and is formulated together with a pharmacologically acceptable carrier.

The pharmaceutical composition of the present invention is a parenteral preparation, which may be a topical preparation directly administered or transplanted at or near an affected site, or a systemic parenteral preparation administered intravenously or intra-arterially. Preferably, the pharmaceutical composition of the present invention is administered intravenously or intra-arterially, preferably intra-arterially, for example, into a carotid artery. Examples of the dosage form include injection preparations such as solution injection preparations, suspension injection preparations, emulsion injection preparations, and ready-to use injection preparations, and implantable preparation. For example, the pharmaceutical composition is formed into an aqueous or nonaqueous isotonic aseptic solution or suspension, and formulated into an appropriate unit dosage form together with a pharmacologically acceptable carrier.

Examples of the pharmacologically acceptable carrier include sterile water, physiological saline, media (in particular, media for use in culturing of mammal cells such as RPMI), physiological buffer solutions such as PBS, vegetable oil, emulsifiers, suspension agents, surfactants, stabilizers, excipients, vehicles, preservatives, and binders. Further, if necessary, the pharmaceutical composition may also contain an isotonic solution containing glucose or other adjuvants, for example, D-sorbitol, D-mannose or D-mannitol, a salt such as sodium chloride, an appropriate solubilizing agent, for example, alcohol, specifically, ethanol, polyalcohol, propylene glycol or polyethylene glycol, a nonionic surfactant, for example, polysorbate 80 or HCO-50, and the like.

When stored in a frozen state, the pharmaceutical composition of the present invention may contain a cryoprotectant such as DMSO, glycerol, polyethylene glycol, propylene glycol, glycerin, polyvinylpyrrolidone, sorbitol, dextran, or trehalose.

The number of hematopoietic stem cells to be incorporated into the pharmaceutical composition of the present invention is appropriately selected according to an age, a body weight, a disease or a symptom of a subject patient, and an administration method. For example, in the case of limb ischemia, the pharmaceutical composition of the present invention is prepared such that the number of hematopoietic stem cells to be administered is about 10⁴ to 10⁶/Kg, preferably 10⁴/Kg or more, more preferably 5 × 10⁴/Kg or more, further more preferably 5 × 10⁵/Kg or more, or 2 × 10⁶/Kg if necessary. In the case of brain infarction, the pharmaceutical composition of the present invention is prepared such that the number of hematopoietic stem cells to be administered is about 10⁴ to 10⁶/Kg, preferably 10⁴/Kg or more, more preferably about 5 × 10⁴/Kg. The total number of cells (mononuclear cells) is about 100 times larger than the above-described numerical value for the hematopoietic stem cells.

### 2.2 Method for treating ischemic disease

The present invention also provides a method for treating an ischemic disease using XR cells. The method comprises parenterally administering or transplanting a population of cells including irradiated hematopoietic stem cells to an ischemic disease patient together with a pharmaceutically acceptable carrier. The population of cells including irradiated hematopoietic stem cells can be prepared by a method described later.

Examples of the "ischemic disease" targeted by the pharmaceutical composition and the treatment method of the present invention include limb ischemia such as lower limb ischemia and Buerger disease; ischemic heart diseases such as myocardial infarction, angina pectoris and cardiac failure; ischemic cerebrovascular disorders such as brain infarction (including atherothrombotic brain infarction, lacunar infarction, BAD and TIA), cerebral thrombus, arterial dissection, venous infarction and vasculitis, vascular dementia, cerebral palsy including neonatal hypoxic ischemic encephalopathy, bilirubin encephalopathy at premature birth, and refractory bone fracture associated with an impaired blood flow (refractory bone fracture incurable because of insufficient blood flow).

Unlike therapies such as stem cell transplantation for age-related macular degeneration, which require engraftment of transplanted cells, in the ischemic diseases, administered cells temporarily remain near the affected site, and it is important to quickly supplement energy substrates (glucose, glutamate, lactate and the like) that are not sufficiently supplied. When administered, the "XR cells" according to the present invention rapidly aggregate near an affected site, and supply necessary energy to cause revascularization and cerebral nerve regeneration.

The "XR cells" according to the present invention comprise hematopoietic stem cells, and are comparable in revascularization promotion ability to non-irradiated cells. That is, the cells according to the present invention maintain cell viability and revascularization promotion ability equivalent to those of non-irradiated cells for 48 hours or more after irradiation, survive for a period necessary for treatment without causing unnecessary immune responses and cell proliferation, and promote the repair of an ischemic site.

### 3. Method for producing pharmaceutical composition for treating ischemic disease and the like

The present invention also provides a method for producing a pharmaceutical composition for treating an ischemic disease and the like, comprising XR cells. The method comprises the steps of: preparing XR cells by, for example, the following method (a) or (b); and formulating the obtained XR cells together with a pharmacologically acceptable carrier if necessary to produce a pharmaceutical composition:
(a) irradiating a sample containing hematopoietic stem cells at 50 Gy or less, and isolating a population of cells including hematopoietic stem cells from the sample, obtaining XR cells, or
(b) isolating a population of cells including hematopoietic stem cells from a sample containing hematopoietic stem cells, and irradiating the population of cells at 30 Gy or less, thereby obtaining XR cells.

The irradiation intensity is, for example, 50 Gy, 45 Gy, 40 Gy, 35 Gy, 30 Gy, 25 Gy, 20 Gy or 18 Gy for the upper limit, and 7.5 Gy, 10 Gy, 12 Gy, 13 Gy, 14 Gy or 15 Gy for the lower limit, and is in the range of, for example, 7.5 to 50 Gy, 10 to 50 Gy, 10 to 30 Gy, 15 to 50 Gy, 15 to 30 Gy or 15 to 25 Gy. The dose is preferably 7.5 to 50 Gy, more preferably 10 Gy to 30 Gy, more preferably 15 to 30 Gy, further more preferably 15 to 25 Gy. For example, irradiation at 15 Gy, preferably an X-ray, is performed.

As the population of cells including hematopoietic stem cells, a population of mononuclear cells or a population of CD34-positive cells may be utilized as described in Section 1, and it is preferable to use a population of CD34-positive mononuclear cells. As the sample containing hematopoietic stem cells, an umbilical cord blood, bone marrow liquid or peripheral blood sample may be used.

### 4. Cell bank for XR cells

The XR cells according to the present invention are safe, have high cell viability, maintain a therapeutic effect of hematopoietic stem cells such as a revascularization promotion effect, and therefore can be incorporated stem cell bank for medical use. The stem cell bank is formed from XR cells formulated together with a cryoprotectant and stored in a frozen state, and supplies XR cells when needed.

### Examples

Hereinafter, the present invention will be described in more detail by way of Examples which should not be construed as limiting the present invention.

### [Example 1]

### Studies on cell viability of irradiated cells

The umbilical cord blood was irradiated at 15 Gy. Thereafter, the umbilical cord blood was mixed with hydroxyethyl starch with a molecular weight of 400,000 (HES40) as an erythrocyte sedimentation promoter, and the mixture was then centrifuged at 50 g for 5 minutes to settle erythrocytic cells, thereby obtaining a leukocyte suspension as supernatant. Next, the leukocyte suspension was overlaid on Ficoll Liquid (manufactured by GE healthcare, specific gravity: 1.077), and centrifugation was performed at 400 g for 30 minutes to obtain mononuclear cells accumulated on the buffy coat. For the non-irradiated cells as a control, the cells were prepared in the same manner as in the case of the irradiated group except that irradiation was not performed.

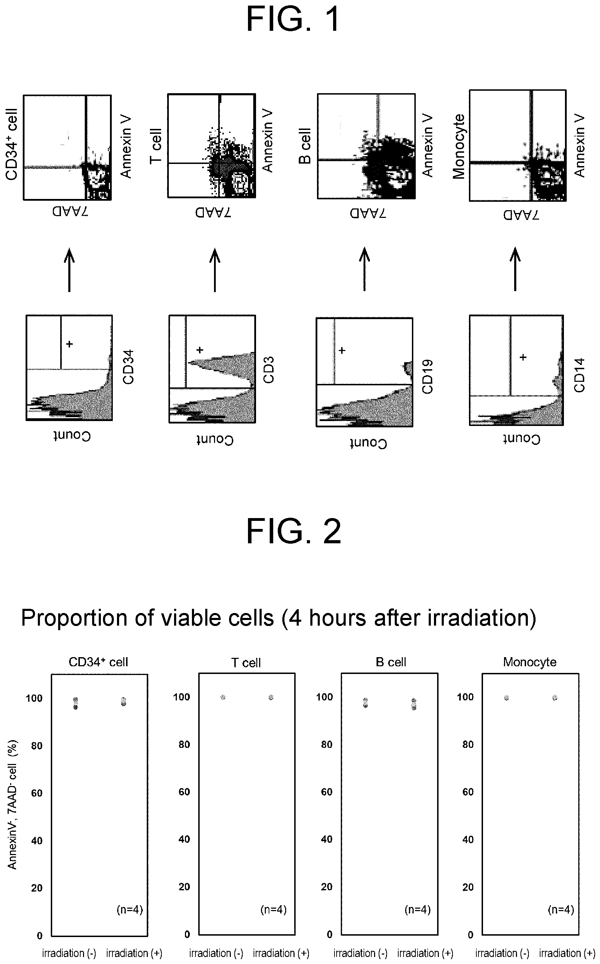

The viability of the cells was assayed in the following manner. With an anti-CD34 antibody (manufactured by BD Bioscience [the same applies for the following], hematopoietic stem cell marker), an anti-CD3 antibody (T cell marker), an anti-CD19 antibody (B cell marker), an anti-CD14 antibody (monocyte marker), Annexin V (early cell death marker) and 7AAD (late cell death marker), the expressions of Annexin V and 7AAD in populations of CD34-positive cells, CD3-positive cells, CD19-positive cells and CD14-positive cells were measured using FACS Canto II Cell Analyzer (BD Bioscience). The results are shown in Figure 1.

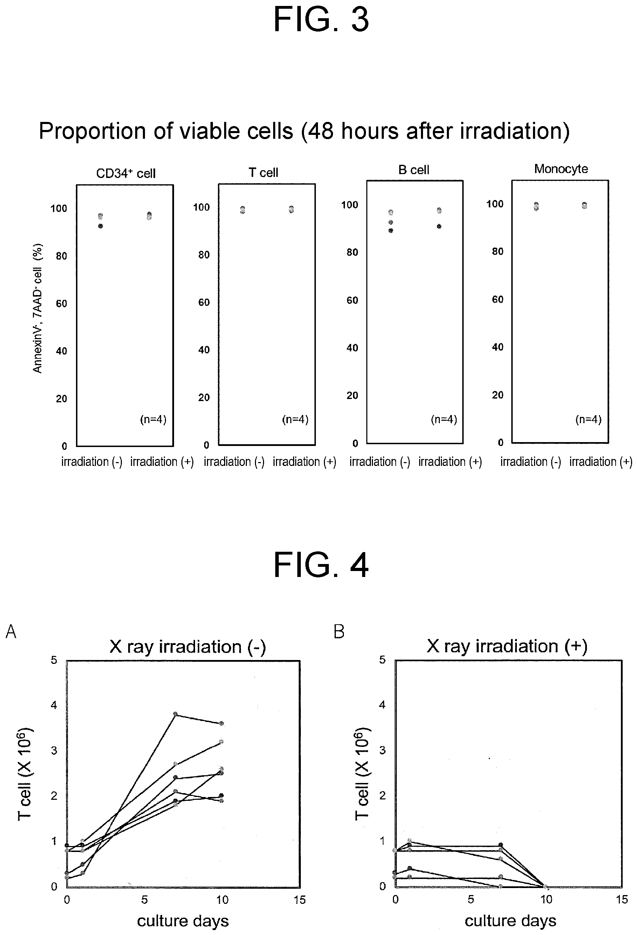

The 7AAD-positive or Annexin V-positive cells in each population of cells were detected, and not only 7AAD-positive cells as a late cell death marker but also Annexin V-positive cells as an early cell death marker were counted as dead cells to determine a proportion of viable cells. The proportions of viable cells 4 hours and 48 hours after the irradiation are shown in Figures 2 and 3, respectively (N = 4 for each group). As shown in these results, the proportion of viable cells in each population of cells was substantially 90% or more 4 hours and 48 hours after the irradiation. This demonstrates that there was no difference in viability between the irradiated and non-irradiated cells.

According the above-listed published report (Perotti et al), umbilical cord blood-derived mononuclear cells irradiated with an X-ray at 70 Gy were used for treatment of lower limb ischemia, and the cell viability 48 hours after irradiation was about 33.5%. For this numerical value, only 7AAD is used for identification of dead cells, and cells that do not express 7AAD, but express Annexin V reflecting an earlier change are counted as viable cells. Therefore, the numerical value of the cell viability would be smaller if counting is performed by the method of the present Example. In contrast, for the irradiated cells prepared by the method of the present Example, not only cells expressing 7AAD but also cells expressing Annexin V were all counted as dead cells, and a viability of 90% or more was achieved as described above.

### [Example 2]

### Studies on possibility of inducing immune reaction by irradiated cells

It is known that when a cell population including allogeneic leucocytes is transplanted, the T cells present in the cell population induce an adverse immune reaction (graft versus host disease; GVHD). In the present Example, a lymphocyte stimulation test was conducted for examining the possibility of inducing an immune reaction by irradiated hematopoietic stem cells. In the present Example, umbilical cord blood-derived mononuclear cells were used as a cell population including hematopoietic stem cells because mononuclear cell fractions contain hematopoietic stem cells in a relatively high ratio.

Cells were prepared in the same manner as in Example 1. That is, the umbilical cord blood was irradiated with an X-ray at 15 Gy. Thereafter, the umbilical cord blood was mixed with hydroxyethyl starch with a molecular weight of 400,000 (HES40) as an erythrocyte sedimentation promoter, and the mixture was then centrifuged at 50 g for 5 minutes to settle erythrocytic cells, thereby obtaining a leukocyte suspension as supernatant. Next, the leukocyte suspension was overlaid on Ficoll Liquid (manufactured by GE healthcare, specific gravity: 1.077), and centrifugation was performed at 400 g for 30 minutes to obtain mononuclear cells accumulated on the buffy coat. To the mononuclear cells, PHA being mitogen was added to a final concentration of 5 µg/mL, and the cells were cultured at 37°C in 5% carbon dioxide. For the non-irradiated cells as a control, the cells were prepared in the same manner as in the case of the irradiated group except that irradiation was not performed.

For the cells, a lymphocyte stimulation test was conducted on the basis of a published report (William FC et al J. Clin Invest 1984). The numbers of T cells on days 1, 7 and 10 with day 0 as start point were used for comparison. The results are shown in Figure 4. The control cells which had not been irradiated with an X-ray proliferated until day 7 or 10, but the cells irradiated with an X-ray were not proliferative at any of the time points, and on day 10, cells died in all samples. That is, it was shown that the irradiated cells prepared by the method of the present Example were not immunoreactive to antigen stimulation, and did not induce an adverse immune inflammatory reaction even after transplantation.

### [Example 3]

### Proliferative and mitotic activity of irradiated cells

Except for patients with blood diseases who are administered with the intention of reconstructing the hematopoietic system with donor cells, it is known that survival, mitosis, and proliferation of donor-derived allogeneic cells in the recipient's body without the control of the recipient's immune cells is extremely dangerous, and often leads to a lethal outcome. In general, donor-derived cells are often rejected if immunity is normal, but it is known that in the case of a patient in an immunodeficient state, in the case of a donor with an HLA type close to that of a recipient, or in the case of a child, the rejection is incomplete, so that unnecessary proliferation and division of donor-derived cells occurs.

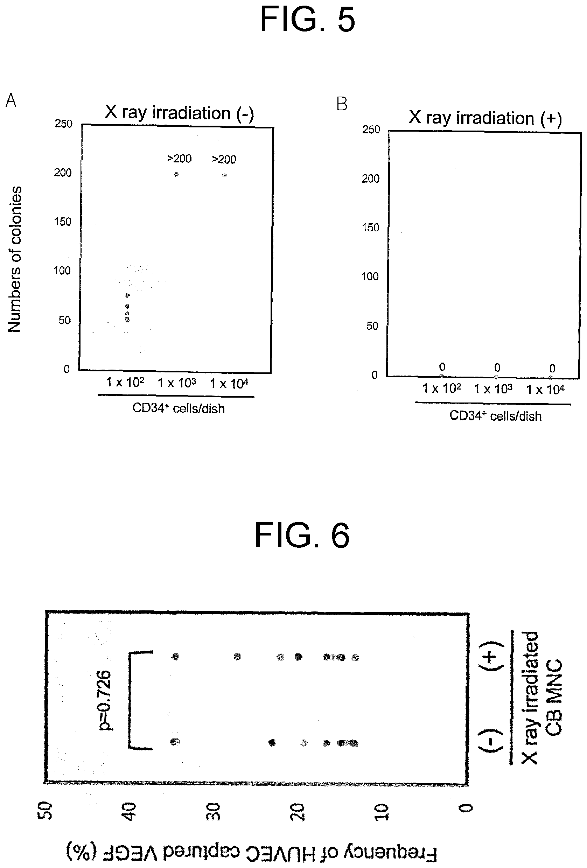

For examining the proliferation and mitotic activity of irradiated cells, a colony formation cell test was conducted on the basis of a published report (Eaves C et al Williams Hematology 5th edition 1995). The separated umbilical cord blood under the same conditions as in Example 1 was irradiated with an X-ray at 15 Gy, mononuclear cells were collected, and the number of CD34-positive cells in the mononuclear fraction was counted. 1 × 10² cells, 1 × 10³ cells and 1 × 10⁴ cells were seeded on media for the colony formation cell test, respectively, and cultured at 37°C in 5% CO₂ for 14 days, followed by measurement of the number of colonies. For the non-irradiated cells as a control, the cells were prepared in the same manner as in the case of the irradiated group except that irradiation was not performed. The results are shown in Figure 5.

The cells which had not been irradiated with an X-ray formed colonies at each concentration, whereas the cells irradiated with an X-ray were not observed to form colonies at any of the cell concentrations. That is, the colony formation cell test showed that the irradiated cells prepared by the method of the present Examples did not have unnecessary proliferation and mitotic activity presenting a major problem associated with transplantation of hematopoietic stem cells, and had no possibility of developing GVHD. Further, it was also shown that the irradiated cells prepared by the method of the present Example died in 7 days, and therefore had no possibility of canceration of transplanted cells.

### [Example 4]

### Studies in vitro on revascularization promotion ability of irradiated cells

The inventors have developed and utilized a method for determining the efficacy of transplanted cells with regard to regenerative medicine for ischemic diseases (Kikuchi-Taura, et al. Stroke 2020 Apr; 51(4): 1279-1289). In the present Example, this method was utilized to examine the revascularization promotion effect of irradiated cells.

In accordance with Example 1, mononuclear cells were separated from the umbilical cord blood, and irradiated cells, and non-irradiated cells as control were prepared. As a fluorescence label VEGF, a streptavidin label VEGF was prepared by incubating biotin-binding VEGF (R&D Systems, Inc.) together with streptavidin-binding APC (Thermo Fisher Scientific, Inc., molar ratio: 4 : 1) at room temperature for 10 minutes. Human umbilical vein endothelial cells (HUVEC, Kurabo Industries Ltd.) were cultured together with a medium (HuMedia-EB2, Kurabo Industries Ltd.), serum, and a growth additive according to the protocols from the manufacturer. Vascular endothelial cells were trypsinized to form a cell suspension at 1 × 10⁵/100 µL, and APC-labeled VEGF (final concentration: 10 nM) were then added to the cells to be measured (1 × 10⁶/50 µL).

The mixture was incubated at 37°C in 5% CO₂ for 3 hours, and the cell mixture was then washed twice with PBS, and stained with a Phycoerythrin (PE)-labeled anti-human CD31 antibody (BD Bioscience), a FITC (Fluorescein isothiocyanate)-labeled anti-human CD45 antibody (BD Bioscience) and 7AAD (BD Bioscience). The level of APC captured in the vascular endothelial cells (CD31-positive, CD45-negative, and 7AAD-negative) was evaluated with FACS Canto II Cell Analyzer (BD Bioscience). The results are shown in Figure 6. The numerical value (Frequency of HUVEC captured VEGF (%)) indicates the "positive ratio" of HUVEC found to be intracellular VEGF (APC)-positive in the histogram after addition of umbilical cord blood cells, that is, the proportion of VEGF-capturing positive cells in HUVEC, which are CD31-positive, CD45-negative, and 7AAD-negative cells.

The irradiated cells were shown to be substantially equivalent to non-irradiated cells with respect to promoting VEGF capture in vascular endothelial cells. This result reviewed together with the results from Examples 2 to 4 may demonstrate that the irradiated cells according to the present invention have no adverse immune response inducing effect, no possibility of causing GVHD, no possibility of proliferating unlimitedly, and are the therapeutic efficacy is comparable to that of non-irradiated cells.

### [Example 5]

### Studies on therapeutic efficacy of irradiated cells for brain infarction

Studies were conducted on therapeutic efficacy of irradiated cells for brain infarction using a brain infarction model mouse.

### 1-1. Preparation of brain infarction model mouse

A brain infarction model mouse was prepared in accordance with a published report (Japanese Patent No. 4481706). Briefly, a 7-week-old severe combined immunodeficient mouse (SCID mouse; CB-17/lcr-scid/scidJcl) was given general anesthesia with isoflurane, and the skull base was perforated to about 1.5 mm so that it was possible to approach from the left zygomatic region and directly reach the left middle cerebral artery. The left middle cerebral artery immediately after passage through the olfactory tract (distal side of the olfactory tract intersection) was clotted using a bipolar electrosurgical knife, and cut after clotting to permanently block the left middle cerebral artery, thereby preparing a brain infarction model limited to the cortex of the left middle cerebral artery region.

### 1-2. Irradiation conditions

The umbilical cord blood was irradiated with an X-ray at 15 Gy as in Example 1. Non-irradiated cells were set as a control. The following steps for preparation of cells to be administered and administration of the cells were the same as those for irradiated cells.

### 1-3. Preparation of cells to be administered Mononuclear cells:

The umbilical cord blood was mixed with hydroxyethyl starch with a molecular weight of 400,000 (HES40) as an erythrocyte sedimentation promoter, and the mixture was then centrifuged at 50×g for 5 minutes to settle erythrocytic cells, thereby obtaining a leukocyte suspension as supernatant. Next, the leukocyte suspension was overlaid on Ficoll Liquid (manufactured by GE healthcare, specific gravity: 1.077), and centrifugation was performed at 400 g for 40 minutes to obtain mononuclear cells accumulated on the buffy coat.

### CD34-Positive cells:

A magnetic bead-labeled anti-CD34-positive antibody was added to the mononuclear cell suspension, and the suspension was passed through a column, thereby isolating CD34-positive cells. For separation, CD34 MicroBead Kit UltraPure (Miltenyi Biotec Company) was used. The cell separation was performed in accordance with the protocol attached to the kit.

### 1-4. Administration of cells

In accordance with a published report, the cells were administered through the left common carotid artery of the brain infarction mouse 48 hours after occlusion of the middle cerebral artery (Kasahara, et al. Transl Res. 2016 Oct; 176: 69-80). The number of administered cells was 1 × 10⁵ for umbilical cord blood mononuclear cells and 1 × 10⁴ for CD34-positive cells. For each type, the cells were suspended in 50 µL of a PBS solution, followed by administration.

### 1-5. Cerebral nerve function test

In the cerebral nerve function test, a non-treated group (no-surgery group, n = 6), a group receiving venous administration of PBS (PBS group, n = 6), a group receiving intra-arterial administration of a mononuclear cell fraction obtained from umbilical cord blood (MNC group, n = 5), a group receiving intra-arterial administration of a mononuclear cell fraction obtained from irradiated umbilical cord blood (MNC-X group, n = 5), a group receiving intra-arterial administration of a CD34-positive cell fraction obtained from umbilical cord blood (CD34⁺ group, n = 5), and a group receiving intra-arterial administration of CD34-positive cell fraction obtained from irradiated umbilical cord blood (CD34⁺-X group, n = 5) were compared.

### 1-5-1. Wire hang test

A wire hang test was conducted as the cerebral nerve function test. In the wire hang test, a mouse was placed at the center of a woven metallic wire of 30 cm × 30 cm having 1 cm-wide meshes. The woven metallic wire was turned upside down, and placed on a supporting stand installed at a height of about 40 cm from a thickly floored open cage. The time until the mouse fell down from the woven metallic wire was measured, and in the case where the mouse remains clinging without falling down, a measured value was set to 180 seconds.

Forty-eight hours after occlusion of the middle cerebral artery, the PBS group and the groups that received intra-arterial administration of a mononuclear cell fraction obtained from umbilical cord blood (MNC), a mononuclear cell fraction obtained from irradiated umbilical cord blood (MNC-X), a CD34-positive cell fraction obtained from umbilical cord blood (CD34⁺), and a CD34-positive cell fraction obtained from irradiated umbilical cord blood (CD34⁺-X) were each compared to the no-surgery group in which brain infarction was not formed.

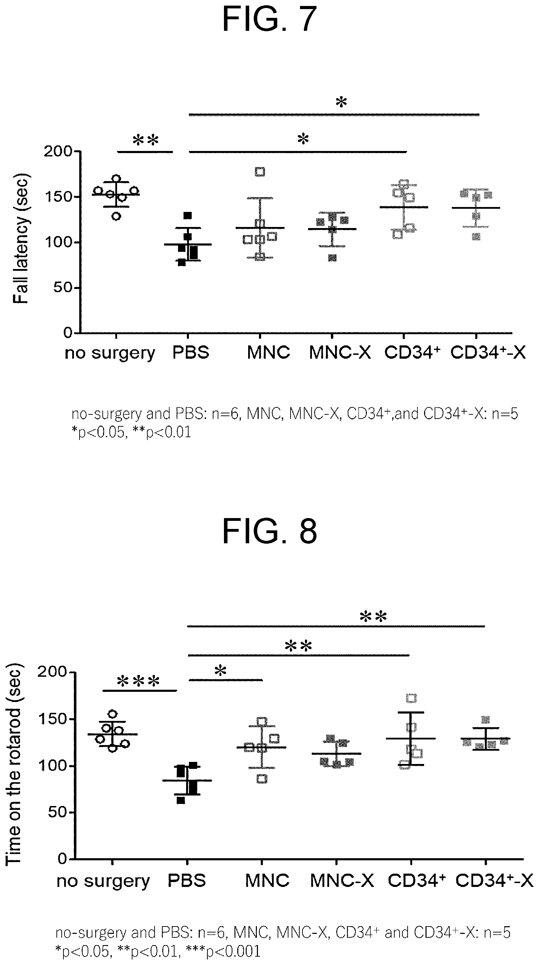

Scores were measured at 12 days after the administration (Figure 7). As shown in Figure 7, the time until the mouse fell down from the woven metallic wire was significantly shorter in the non-treated group in which brain infarction was formed (PBS group) than in the no-surgery group. That is, there was a significant decrease in motor function in the PBS group as compared to the no-surgery group. On the other hand, for the cell administration groups, there was a significant improvement in motor function in the CD34⁺ and CD34⁺-X groups as compared to the PBS group. The motor function tended to be improved in MNC and MNC-X although there was no statistically significant difference. Comparison between MNC and MNC-X and between CD34⁺ and CD34⁺-X did not show a significant difference.

From the above results, it has become evident that intra-arterial administration of an umbilical cord blood-derived mononuclear cell fraction and a CD34-positive cell fraction 48 hours after occlusion of the middle cerebral artery (subacute phase) has therapeutic efficacy for impairment of motor function after brain infarction. In addition, it has been found that even if irradiation is performed, the cerebral nerve function improvement effect of the cells is not influenced.

### 1-5-2. Rotarod test

As a cerebral nerve function test, a rotarod test was adopted. The rotarod test is intended to measure the coordinated movement and the balance sense of a mouse. The apparatus used is MK630A (Muromachi Kikai Co., Ltd.). The apparatus was programmed to accelerate the rotation of a rod to 4 rpm to 40 rpm in 300 seconds, and the time (sec) until a mouse fell down from a rotation bar after being placed thereon was recorded.

Forty-eight hours after occlusion of the middle cerebral artery, the PBS group and the groups that received intra-arterial administration of a mononuclear cell fraction obtained from umbilical cord blood (MNC), a mononuclear cell fraction obtained from irradiated umbilical cord blood (MNC-X), a CD34-positive cell fraction obtained from umbilical cord blood (CD34⁺), and a CD34-positive cell fraction obtained from irradiated umbilical cord blood (CD34⁺-X) were compared to the no-surgery group in which brain infarction was not formed.

Scores were measured at 14 days after the administration (Figure 8). As shown in Figure 8, the time until the mouse fell down from the rotation bar was significantly shorter in the non-treated group in which brain infarction was formed (PBS group) than in the no-surgery group. That is, there was a significant decrease in motor function in the PBS group as compared to the no-surgery group. However, there was a statistically significant improvement in motor function in MNC, CD34⁺ and CD34⁺-X groups receiving administration of the cells as compared the PBS group. The function tended to be improved in the MNC-X group as compared to the PBS group although there was no statistically significant difference. Comparison between MNC and MNC-X and between CD34⁺ and CD34⁺-X did not show a significant difference.

From the above results, it has become evident that intra-arterial administration of an umbilical cord blood-derived mononuclear cell fraction and a CD34-positive cell fraction 48 hours after occlusion of the middle cerebral artery (subacute phase) has therapeutic efficacy for impairment of motor function after brain infarction. In addition, it has been found that even if irradiation is performed, the cerebral nerve function improvement effect of the cells is not influenced.

### 1-5-3. Open field test

As a cerebral nerve function test, an open field test was adopted. The open field test is intended to measure the locomotor activity of a mouse. In a rodent brain infarction model, a significant decrease in locomotor activity has been recognized 14 to 18 days after preparation of the model, which is considered to reflect a depressed state after brain infarction. The amount of locomotor activity of a mouse was measured using an open field apparatus from Taiyo Electric Co., LTD. In this apparatus, infrared beams are mounted at regular intervals on X, Y and Z axes of an open field box (30 × 30 × 30 cm), and the total number of beam interrupts is counted as a behavior of the mouse. The mouse was allowed to freely act in the box for a total of 60 minutes in an illuminated light environment for first 30 minutes and in an unlit state for subsequent 30 minutes, and the amount of action was measured.

The no-surgery group in which brain infarction was not formed was compared to the PBS group and each of the groups that received intra-arterial administration of a mononuclear cell fraction obtained from umbilical cord blood (MNC), a mononuclear cell fraction obtained from irradiated umbilical cord blood (MNC-X), a CD34-positive cell fraction obtained from umbilical cord blood (CD34⁺), and a CD34-positive cell fraction obtained from irradiated umbilical cord blood (CD34⁺-X) 48 hours after occlusion of the middle cerebral artery.

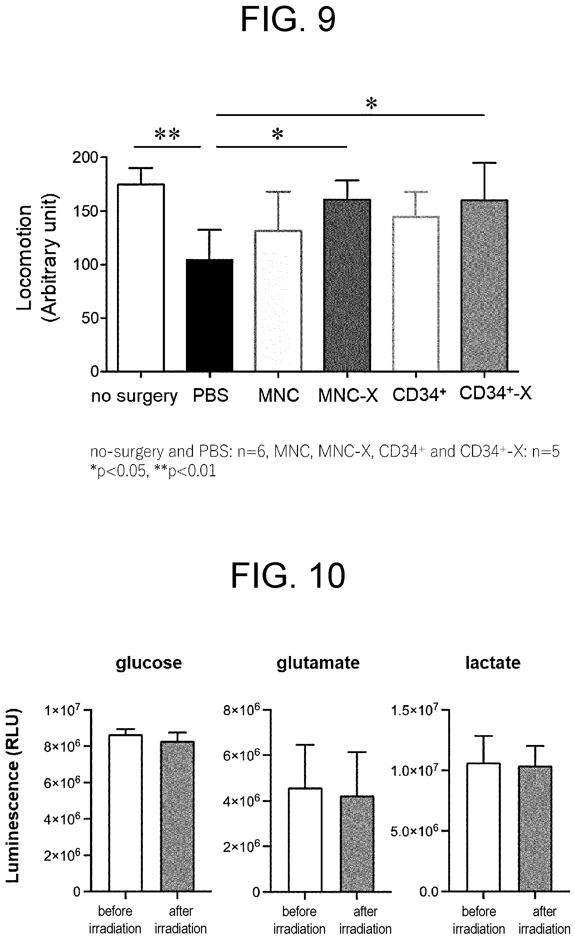

Scores were measured at 15 to 17 days after the administration (Figure 9). As shown in Figure 9, the amount of horizontal movement in the active phase (dark phase) in the group which had not been treated after brain infarction (PBS group) significantly decreased as compared to that in the no-surgery group. On the other hand, in the MNC-X group and the CD34⁺-X group, among the groups receiving administration of cells, the amount of action increased with a statistically significant difference shown as compared to the PBS group. In the MNC and CD34⁺ groups, the amount of action tended to increase as compared to the PBS group although there was no statistically significant difference, and a statistically significant difference with respect to the no-surgery group like that observed between the PBS group and the no-surgery group disappeared.

From the above results, it has become evident that the intra-arterial administration of an umbilical cord blood-derived mononuclear cell fraction and a CD34-positive cell fraction 48 hours after occlusion of the middle cerebral artery (subacute phase) has therapeutic efficacy for a decrease in the amount of action which is associated with a depressed state occurring after brain infarction. In addition, it has been found that even if irradiation is performed, therapeutic efficacy of the cells is not influenced.

### [Example 6]

### Influence of irradiation on low-molecular-weight energy sources in cells

An influence of irradiations on low-molecular-weight energy sources in cells was examined.

As in Example 1, the umbilical cord blood was irradiated with an X-ray at 15 Gy, immediately followed by cell separation. Cells obtained by isolating a portion of the umbilical cord blood before the irradiation, and performing separation in the same manner as in Example 1 were set as cells before irradiation, and used for comparison before and after the irradiation. After the cell separation, a cryoprotectant solution (CB045; Stem Cell Banker GMP grade, Nippon Zenyaku Kogyo Co., Ltd.) was added to all the cells, followed by storage in liquid nitrogen. The cells were thawed immediately before measurement, and used for experiments. Immediately after the thawing, the concentrations of glucose, glutamate, and lactate as energy sources for the cells were measured using Glucose-Glo^{™} Assay, Glutamate-Glo^{™} Assay and Lactate-Glo^{™} Assay Kits manufactured by Promega Corporation in accordance with the respective protocols. The number of cells used was 1 × 10⁵ per assay (n = 3).

The results showing that there is no difference in concentration of substances as energy sources before and after irradiation as shown in Figure 10 are consistent with the results showing that there is no difference in therapeutic efficacy before and after irradiation in the *in vivo* test.

### [Example 7]

### Lymphocyte stimulation test with CD3/CD28 antigen

The whole umbilical cord blood was equally divided, and only one part of the whole blood was irradiated (with an X-ray at 15 Gy). Mononuclear cells were separated from the whole umbilical cord blood by a specific gravity centrifugation method, and T cells were separated from the mononuclear cell fraction using the Human Pan T Cell Separation Kit (Miltenyi Biotec). Specifically, the umbilical cord blood-derived mononuclear cells were suspended in PBS-BSA-EDTA at 1 × 10⁷ cells per 40 µL, and to this, 10 µL of an antibody cocktail containing a biotin-bound monoclonal antibody (antibodies against CD14, CD15, CD16, CD19, CD34, CD36, CD56, CD123 and CD235a) was added, followed by incubation in a refrigerator (2 to 8°C) for 5 minutes. Next, 30 µL of PBS-BSA-EDTA was added, 10 µL of avidin-bound magnetic beads were added, and the mixture was incubated in the refrigerator for 10 minutes. The cell suspension was washed with PBS-BSA-EDTA, and then filtered through a 30 µm nylon mesh, and a passing fraction was collected using a column installed on a magnetic field. The purity of CD³⁺ T cells obtained after the purification process was carried out twice was 95% or more.

The isolated T cells were co-cultured in RPMI 1640 medium (containing 10% FBS) with Dynabeads coated with an anti-CD3/CD28 antibody (Dynabeads Human T-Activator CD3/CD28, Thermo Fisher Scientific, Inc.). The amount of Dynabeads added to 1 × 10⁷ T cells was 5 µl. Further, IL-2 (Chiron, Emeryville, CA) was added to a final concentration of 100 µg/mL on day 0, and the cells were subcultured every three days in a rapid proliferation phase.

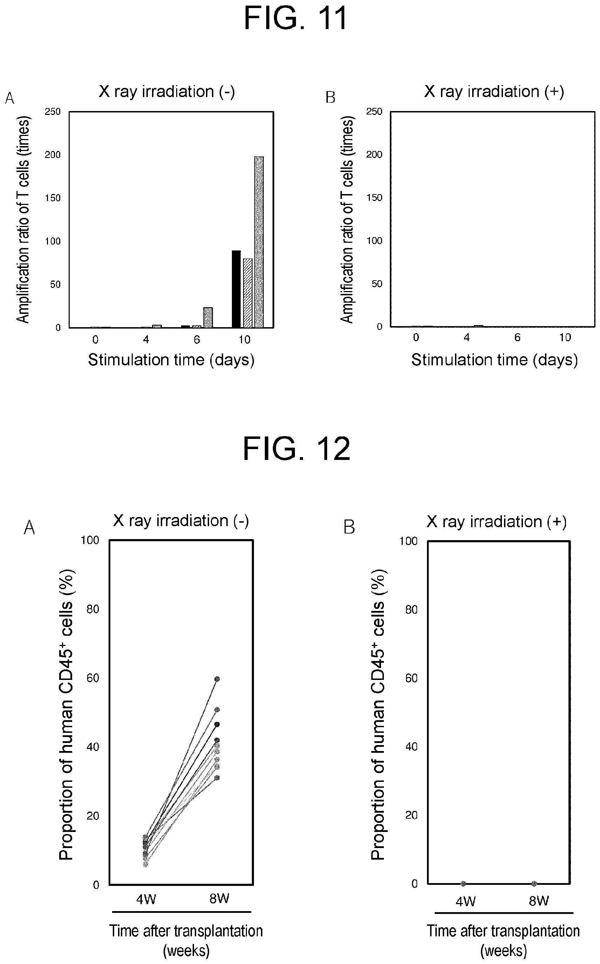

As a result, the umbilical cord blood T cells which had not been irradiated (with an X-ray) proliferated rapidly on the sixth and subsequent days after the stimulation, with the amplification ratio reaching 122 times (n = 3) on day 10 (Figure 11A), whereas the irradiated umbilical cord blood T cells did not proliferate at all even on very strong stimulation with a CD3/CD28 antigen (Figure 11B). These results suggest that irradiated cells are not immunoreactive to antigen stimulation, and have little risk of GVDH.

### [Example 8]

### Data on safety of irradiated cells

From Central Institute for Experimental Animals (Kawasaki, Japan), 5-week-old NOD. Cg-PrkdcscidIl2rgtm1 Sug/ShiJic (NOG/SCID) mice were obtained. All the mice were reared in a sterile condition, and the experiments were conducted in a sterile isolator. The 20 NOG/SCID mice were divided into two groups. For one group as a positive control, 5 × 10⁴ umbilical cord blood CD34⁺ cells which had not been irradiated were transplanted through tail vein injection into NOG/SCID mice sub-lethally irradiated (with an X-ray) at 2 Gy. For the other 10 mice, 1 × 10⁶ umbilical cord blood CD34⁺ cells irradiated (with an X-ray) at 15 Gy were also transplanted into NOG/SCID mice. After 4 and 8 weeks, the proportion of human CD45⁺ cells in the transplanted mouse peripheral blood was analyzed by flow cytometry.

Where a case in which 0.1% or more of the whole mouse peripheral blood or BM cells are human CD45⁺ cells is defined as engraftment, engraftment was observed in all of the mice undergoing transplantation of umbilical cord blood CD34⁺ cells which had not been irradiated, and the average positive ratios were 10.7% and 41.4% (Figure 12A). On the other hand, when irradiated umbilical cord blood CD34⁺ cells were transplanted, engraftment was not observed in any of the mice although the amount of CD34⁺ cells transplanted was increased by 20 times, and the average positive ratio was 0.0% or less (Figure 12B). These results suggest that the irradiated umbilical cord blood is extremely low not only in risk of GVHD but also in risk of tumor formation.

### [Example 9]

### Studies on apoptosis of irradiated cells

The umbilical cord blood was equally divided, and only one part was irradiated (with an X-ray) at 15 Gy. From the irradiated umbilical cord blood and the umbilical cord blood which had not been irradiated, mononuclear cells were separated by a specific gravity centrifugation method, and T cells and CD34⁺ cells were separated using Human Pan T Cell Separation Kit (Miltenyi Biotec) and CD34-Positive Cell Separation Kit (Miltenyi Biotec), respectively. For proliferation stimulation, the T cells were stimulated in RPMI medium for 24 hours with Dynabeads coated with an anti-CD3/CD28 antibody, and IL-2, and the CD34⁺ cells were cultured for 24 hours in IMDM medium containing various hematopoietic cytokines (EPO, IL-3, G-CSF and GM-CSF). The kinetics of apoptotic cells in the cultured T cells and CD34⁺ cells were measured by double staining using FITC-labeled Annexin V (nacalai tesque, Kyoto, Japan) and 7AAD (Becton, Dickinson and Company).

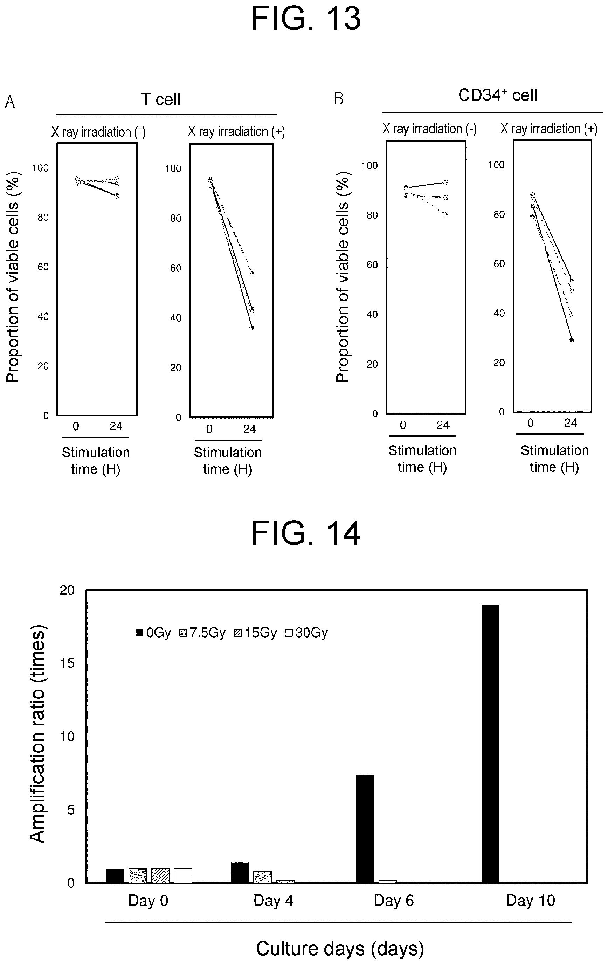

When proliferation stimulation was not performed, the proportion of apoptotic cells in the T cells and CD34⁺ cells was as low as 10% or less regardless of whether they were irradiated or not (Figure 13). On the other hand, the proportion of apoptotic cells after proliferation stimulation was 10% or less and equivalent to that before proliferation stimulation when the irradiation was not performed, but the proportion of apoptotic cells in the T cells and CD34⁺ cells after proliferation stimulation increased to about 50% when the irradiation was performed. These results show that T cells and CD34⁺ cells do not die at the time of performing irradiation, but die only when proliferation signals enter.

### [Example 10]

### Studies on dose of radiation

The whole umbilical cord blood was equally divided into 4 parts, and irradiated with an X-ray at 0 Gy, 7.5 Gy, 15 Gy and 30 Gy. From each part, mononuclear cells were separated by a specific gravity centrifugation method, and from the mononuclear cell fraction, T lymphocytes were purified using Human Pan T Cell Separation Kit (Miltenyi Biotech). Anti-CD3/CD28 antibody-bound beads and IL-2 were added to the purified T lymphocytes, and the cells were cultured for 10 days. The number of cells was measured on culture days 0, 4, 6 and 10, and the results showed that the T cells of the umbilical cord blood which had not been irradiated proliferated significantly, but whereas cells from the umbilical cord blood irradiated at 7.5 Gy or more did not proliferate at all (Figure 14).

### Industrial Applicability

The population of cells including irradiated hematopoietic stem cells (XR cells) according to the present invention maintains cell viability of 90% or more even 48 hours after irradiation, does not either induce an unnecessary immune reaction or have proliferative ability, and has revascularization promotion activity equivalent to that of non-irradiated cells for the period necessary for treatment. Therefore, the present invention is useful for treatment of ischemic diseases and the like.

All publications, patents and patent applications cited herein are directly incorporated herein by reference.

## Claims

1. A pharmaceutical composition for treating an ischemic disease, comprising a population of cells including irradiated hematopoietic stem cells, wherein the irradiation is irradiation at 50 Gy or less.

2. The pharmaceutical composition according to claim 1, wherein the population of cells has cell viability of 90% or more even 3 hours after the irradiation.

3. The pharmaceutical composition according to claim 1, wherein the population of cells including hematopoietic stem cells is a population of mononuclear cells.

4. The pharmaceutical composition according to claim 1, wherein the population of cells including hematopoietic stem cells is a population of CD34-positive cells.

5. The pharmaceutical composition according to claim 1, wherein the population of cells including hematopoietic stem cells is derived from umbilical cord blood, bone marrow liquid or peripheral blood.

6. The pharmaceutical composition according to claim 1, wherein the irradiation is irradiation at 7.5 Gy to 30 Gy.

7. The pharmaceutical composition according to any one of claims 1 to 6, wherein the ischemic disease is one selected from the group consisting of limb ischemia, myocardial infarction, angina pectoris, cardiac failure, brain infarction, arterial dissection, venous infarction, vasculitis, vascular dementia, cerebral palsy including neonatal hypoxic ischemic encephalopathy, bilirubin encephalopathy at premature birth, and refractory bone fracture associated with an impaired blood flow.

8. The pharmaceutical composition according to claim 7, wherein the ischemic disease is brain infarction.

9. A method for producing a pharmaceutical composition for treating an ischemic disease, the method comprising the steps of:
1) preparing a population of cells including irradiated hematopoietic stem cells by one of the following methods (a) and (b):
(a) irradiating a sample containing hematopoietic stem cells at 50 Gy or less, and isolating a population of cells including hematopoietic stem cells from the sample, and
(b) isolating a population of cells including hematopoietic stem cells from a sample containing hematopoietic stem cells, and irradiating the population of cells at 50 Gy or less; and
2) producing a pharmaceutical composition comprising the population of cells including irradiated hematopoietic stem cells.

10. The method according to claim 9, wherein the irradiation is performed at 7.5 Gy to 30 Gy.

11. The method according to claim 9, wherein the population of cells including hematopoietic stem cells is a population of mononuclear cells.

12. The method according to claim 9, wherein the population of cells including hematopoietic stem cells is a population of CD34-positive cells.

13. The method according to claim 9, wherein the population of cells including hematopoietic stem cells is derived from umbilical cord blood, bone marrow liquid or peripheral blood.

14. The method according to any one of claims 9 to 13, comprising the step of adding a cryoprotectant to the pharmaceutical composition, followed by freezing.
